# EUROPÄISCHE PATENTANMELDUNG

(11) **EP 2 282 196 A1**
(43) Veröffentlichungstag der Anmeldung: **09.02.2011**
(21) Anmeldenummer: 10169943.7
(22) Anmeldetag: 19.07.2010
(51) Int. Cl.: G01N 21/898, G01N 33/46

(54) **Verfahren zur Erkennung von Bläue bei Holz**

(30) Priorität: 06.08.2009 AT 12382009
(71) Anmelder: Stora Enso Wood Products GmbH, 3531 Brand (AT)
(72) Erfinder: Prodinger, Karl, 3542 Gföhl (AT); Kraus, Franz, 3324 Euratsfeld (AT); Bicker, Andreas, 3250 Wieselburg (AT)
(74) Vertreter: Babeluk, Michael

(57) **Zusammenfassung**

Die Erfindung betrifft ein Verfahren zur Erkennung von Bläue bei Holz an einer Schnittfläche (2) eines Stammes (1), wobei von der Schnittfläche (2) reflektiertes Licht von zumindest einer bildgebenden Kamera (3) erfasst wird. Um einfach und zuverlässig Blaufäule bei Holz erkennen zu können, ist vorgesehen, dass zumindest zwei Bilder in zumindest einem ersten und einem zweiten Wellenlängenbereich des elektromagnetischen Spektrums erfasst und die Bilder verglichen und ausgewertet werden.

## Beschreibung

Die Erfindung betrifft ein Verfahren zur Erkennung von Bläue bei Holz an einer Schnittfläche eines Stammes, wobei von der Schnittfläche reflektiertes Licht von zumindest einer bildgebenden Kamera erfasst wird. Weiters betrifft die Erfindung eine Vorrichtung zur Erkennung von Bläue bei Holz an einer Schnittfläche eines Stammes, mit zumindest einer bildgebenden Kamera zur Erfassung von der Schnittfläche reflektiertem Licht.

Bei Rundholz tritt nach längerer Lagerung häufig ein Befall mit Bläuepilzen auf, die das Nutzholz in Richtung einer grauen bis bläulichen Verfärbung verändern. Ein durch Bläue befallenes Holz gilt als minderwertig und erzielt auf dem Markt einen wesentlich geringeren Preis. Daher ist es für einen Schnittholzverarbeiter von größtem Interesse, das Ausmaß und die Lage der Bläue zu erkennen, um einen großen Anteil des nutzbaren Holzes gewinnen zu können. Bei der Verarbeitung des Rundholzes vom Lagerplatz weg sind die Flächen des Rundholzstammes in der Praxis gelegentlich verschmutzt, so dass Verfärbungen von außen nicht erkennbar sind. Erst beim Reinigungsschnitt, dem ersten Kappen der verschmutzten Enden des Rundholzes, kann die Bläue als eine, von außen nach innen wachsende, unregelmäßige Verfärbung, erkannt werden. Die Verfärbungen sind an der Schnittfläche in der Regel nur schwach erkennbar, der Sortierer, der die Stämme nach ihrer Qualität beurteilt, ist kaum in der Lage, die Farbveränderungen zu erkennen. Daher ist es wünschenswert, ein praxisgerechtes System zu entwickeln, das zumindest auf einem Bildschirm die Bläue kontrastreich darstellt oder in einer weiteren Entwicklungsstufe eine selbständige Sortierung vornehmen kann.

Verfahren zum Beurteilen der Farbe von Holz sind schon lange bekannt und es wurden zum Teil sehr aufwändige Systeme entworfen, wie aus der DE 10 2006 044 307 A1, der US 2006/0056659 A1 oder der US 7,304.740 B1 hervorgeht. Alle drei Schriften beschreiben Auswerteverfahren für das durchlaufende Holz nach dem Aufschnitt, dem Zerlegen des Stammes in einzelne Bretter, um die von der Bläue befallenen Teile zu erkennen und auszusortieren. Die Verfahren benötigen eine Oberfläche des Holzes mit geringer Rauhigkeit, annähernd die Qualität nach dem Hobeln. Aufgrund der gewählten Beleuchtungstechniken mit Seitenlicht in den genannten Schriften entstehen beim rohen Schnitt an der Stirnseite erhebliche Schatten, die eine Beurteilung auf Bläue wesentlich erschweren.

Zur Auswertung werden bei den bekannten Verfahren im allgemeinen Zeilenkameras verwendet, die auf bestimmte Bereiche des sichtbaren Lichts bis in das angrenzende Infrarot eingestellt sind. Durch die Verwendung von drei Kameras ergeben sich sehr große Aufwendungen bezüglich der Berechnung der Bildinhalte, da sowohl der räumliche und damit zeitliche Versatz, als auch die unterschiedlichen Helligkeiten in einem aufwändigen Verfahren rechnerisch ausgeglichen werden müssen. Die Schattenbildung bei der Beurteilung von Hirnholzstücken verhindert bei den Verfahren eine brauchbare Aussage, die Oberflächen müssen gehobelt werden. Die vorgestellten Systeme sind durchwegs stationär und in den Schnittbereich eingebaut.

Aufgabe der Erfindung ist es, diese Nachteile zu vermeiden und eine zuverlässige und einfaches Erkennen von Bläue zu ermöglichen.

Erfindungsgemäß wird dies dadurch erreicht, dass zumindest zwei Bilder in zumindest einem ersten und einem zweiten Wellenlängenbereich des elektromagnetischen Spektrums erfasst und die Bilder verglichen und ausgewertet werden, wobei vorzugsweise der erste Wellenlängenbereich elektromagnetische Strahlung im für Menschen sichtbaren Bereich, vorzugsweise im blauen und/oder grünen Bereich des Lichtspektrums, und der zweite Wellenlängenbereich elektromagnetische Strahlung im Infrarotbereich abdeckt.

Bei Versuchen hat sich heraus gestellt, dass die Bläue bei senkrechter, schattenfreier Bestrahlung der Schnittfläche mit Infrarotlicht und einer Auswertung über eine gewöhnliche Schwarz-Weiß CCD Kamera (SW Kamera) sehr kontrastreich darstellbar ist, während dunkle Teile wie Rinde oder Äste kaum in Erscheinung treten. Der Pilz, der die Blaufärbung verursacht, absorbiert Infrarotlicht wesentlich stärker als das gesunde Holz. Selbst geringe Schattierungen, die bislang als Grenzfall einzustufen waren, sind mit Infrarot einwandfrei erkennbar. Durch die von allen Seiten gleiche Beleuchtung, im Gegensatz zu den bislang üblichen Techniken, wird die Entstehung von Schatten vermieden und es können die beim Querschnitt üblichen Vertiefungen zwischen den Jahresringen voll ausgeleuchtet und damit ausgewertet werden. Der bislang erforderliche Zwischenschritt mit dem Hobeln der Anschnittfläche kann entfallen, das Ergebnis ändert sich in keiner Weise.

Zur sicheren Darstellung der Bläue wurde eine Schwarz-Weiß CCD-Kamera verwendet, die von sich aus eine hohe Empfindlichkeit sowohl im sichtbaren, als auch nahen Infrarotbereich hat. Diese Kameras werden z.B. für die Überwachung von Objekten verwendet, um eine unbemerkte Kontrolle auf Personen, bei Eingängen etc. durchzuführen und besitzen mehrere Infrarot-Leuchtdioden als Lichtquelle. Die Leuchtdioden haben eine Wellenlänge zwischen 800 nm bis 1.000 nm und sind rund um die Kamera angeordnet. Die vorhandenen Leuchtdioden wurden ergänzt mit mehreren blau oder grün leuchtenden Leuchtdioden. Diese Farben wurden ausgewählt, da sie einen geringeren Kontrast der Bläue gegenüber dem gesunden Holz abgeben.

Werden die beiden Bilder in einem System zum Aufbereiten von Video-Signalen abgespeichert, dann lässt sich die Differenz aus beiden Bildern bestimmen. Als Ergebnis wird die Bläue sehr kontrastreich dargestellt, während die natürlichen Einfärbungen des Holzes durch Jahresringe, Äste etc. wegfallen. Aufgrund der weitgehend bereinigten Darstellung der Bläue und der gesamten Fläche des Anschnittes vom Reinigungsschnitt her kann der Anteil der Bläue prozentuell bestimmt werden und diese Zahl als Kriterium für die Sortierung verwendet werden.

Um die gefundenen Ergebnisse auch in der Praxis nutzbar zu machen, sind zwei Verfahren zur Feststellung von Bläue möglich:
1. Beleuchten der Schnittfläche mit abwechselnd einem Licht im sichtbaren Bereich, wie weißes Licht oder farbiges Licht mit einem geringen bis keinen Anteil an Rot oder Gelb und als zweite Farbe rotes bis infrarotes Licht, da hier die Kontraste besser aufgelöst werden.
2. Nutzen des Umgebungslichtes, wenn alle Farbanteile vorhanden sind, wobei die Lichtquelle sowohl natürlichen, als auch künstlichen Ursprungs sein kann. Die Zerlegung des Lichtes, das von der Schnittstelle zurück geworfen wird, erfolgt anschließend im Bereich der Kamera, durch Filter. Die Filter können, je nach Anordnung, für den gewünschten Bereich absorbierend oder durchlässig sein. Es ist auch möglich, mit Hilfe von wellenlängensensitiven, halbdurchlässigen Spiegeln und mindestens zwei Kamerasystemen eine Aufbereitung des Lichtes durchzuführen.

Natürlich kann auch eine Kombination beider Verfahren vorgenommen werden, um bei wechselnden Lichtverhältnissen ein stabiles Bild zu bekommen.

Die Erfindung wird im folgenden an Hand der Figuren näher erläutert. Es zeigen:
- Fig. 1: eine erfindungsgemäße Vorrichtung in einer ersten Ausführungsva- riante;
- Fig. 2: eine erfindungsgemäße Vorrichtung in einer zweiten Ausführungs- variante; und
- Fig. 3: eine erfindungsgemäße Vorrichtung in einer dritten Ausführungsva- riante.

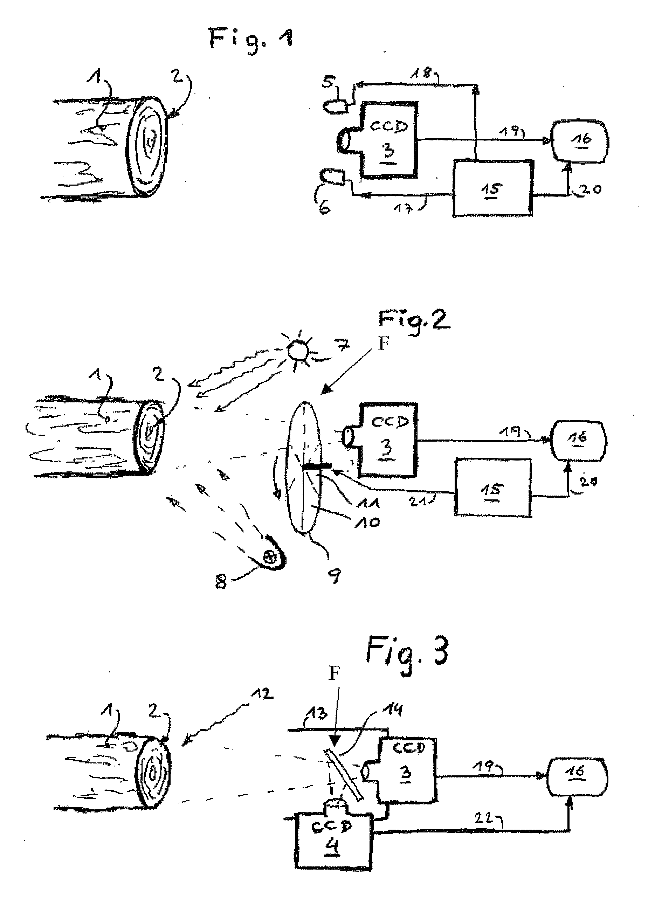

Fig. 1 zeigt den Aufbau der Anordnung mit Eigenlicht und ohne Filter. Der frisch geschnittene Stamm 1 weist mit der Schnittfläche 2 vom Reinigungsschnitt in Richtung zur Kamera 3. Beidseitig der Kamera 3 befinden sich Strahlungsquellen 5 und 6 für weißes oder farbiges Licht und für Infrarotlicht. Vorzugsweise werden hier Leuchtdioden der betreffenden Farbe, z.B. blau oder grün und Infrarot verwendet, um der Kamera 3 von vornherein die richtige Lichtqualität anzubieten. Ein Steuergerät 15 bewirkt das zeitversetzte Einschalten der Strahlungsquellen 5 und 6 über die jeweiligen Leitungen 17 und 18. Über die Leitung 19 wird das Videosignal von der Kamera zur Auswerteeinheit 16 geleitet, das Steuersignal 20 teilt dem Auswertesystem mit, welche Strahlungsquelle 5, 6 gerade eingeschaltet ist.

Eine Schwäche der Anordnung besteht darin, dass bei Fremdlicht (Tageslicht, Raumbeleuchtung) die Auswertung schlechter wird.

Fig. 2 zeigt beispielhaft eine Lösung, die mit dem Umgebungslicht, zum Beispiel dem Tageslicht 7 oder Kunstlicht 8 arbeitet. Um die notwendigen Wellenlängen auswerten zu können, wird eine Filtereinrichtung F mit wechselnden Filtern, hier dargestellt als Filterrad 9 verwendet. Das Filterrad 9 weist mindestens 2 unterschiedliche Filter 10 und 11 bildende Segmente auf, die entweder für das gewünschte blaue oder grüne Bild durchlässig sind oder für Infrarot. Die Anordnung wird vom Steuergerät 15 koordiniert, welches einerseits über die Leitung 21 den jeweiligen Filter vorschaltet und andrerseits der Auswerteeinheit 16 über die Leitung 20 mitteilt, welcher Filter vorgeschaltet ist. Die Auswerteeinheit 16 wird über die Leitung 19 mit dem Videosignal von der Kamera 3 versorgt. Die zusätzlichen Lichtquellen 5 und 6 aus Fig. 1 können ebenfalls im System enthalten sein, sie werden hier aber nicht mehr geschaltet.

Die Anordnung hat den Vorteil, dass jedes Umgebungslicht, das auch einen Anteil im nahen Infrarot aufweist, für die Auswertung herangezogen werden kann. Nachteilig ist das Filterrad als empfindliches, bewegliches Teil, das bei längerem Gebrauch in rauer Umgebung Anlass für Störungen sein kann und das auch in der Herstellung relativ aufwändig und somit teuer ist.

Mit der Fig. 3 wird eine weitere Lösung dargestellt, die ebenfalls mit dem Umgebungslicht 12 arbeitet. Für die Fig. 3 gelten dieselben Lichtquellen wie in Fig. 2, mit den Bezeichnungen 7 und 8. Durch das Umgebungslicht wird wieder der Stamm 1 mit der Schnittfläche 2 beleuchtet, wobei durch nicht dargestellte Abschattungen oder reflektierende Flächen eine gleichmäßige Ausleuchtung erreicht werden soll. Die Anordnung mit zwei Kameras 3 und 4 wird in einem Tubus 13 untergebracht, der die Anlage gegen Fremdlicht von außen schützt. Im Tubus 13 befindet sich ein selektiv beschichteter Spiegel 14, der in einem Winkel von 45 Grad zur Bildachse der Kamera 3 angeordnet ist. Dieser Spiegel 14 hat die Eigenschaft, infrarotes Licht gerade durchzulassen, während der sichtbare Anteil des Lichts um 90 Grad reflektiert wird. Das reflektierte Licht vom Spiegel 14 wird von der Kamera 4 aufgenommen und liefert den sichtbaren Anteil. Durch weitere, hier nicht dargestellte Filter für z.B. blau oder Infrarot kann die Trennschärfe weiter erhöht werden. Vergleichbare Spiegel sind in größerer Anzahl in den bekannten Overhead-Projektoren zum Ausblenden des Wärmeanteils von der Lichtquelle verwendet worden, ebenso in den heute üblichen Beamern. Über die Leitungen 19 und 22 werden beide Videosignale ständig zur Auswerteeinheit 16 geleitet, die Einheit 16 entscheidet selbst, welches Signal sie gerade verarbeitet. Das Steuergerät 15 entfällt. Die Verwendung von zwei Schwarz-Weiß-Kameras 3, 4 bedeutet bei den heutigen Kosten keine besondere Belastung, sie liegen in der Größenordnung des nicht mehr notwendigen Taktgebers.

Die zusätzlichen Lichtquellen 5 und 6 aus Fig. 1 können auch im System enthalten sein, sie werden hier ebenfalls nicht mehr geschaltet.

Ein besonderer Vorteil dieser Anordnung nach Fig. 3 besteht in der Auswertung des reflektierten Umgebungslichtes, mit der Option, bei schlechten Lichtverhältnissen die eigenen Lichtquellen zuschalten zu können. Die einfachste Auswerteeinheit besteht aus der Kameraeinheit und einem handelsüblichen Bildschirm, der z.B. beide Bilder neben- oder hintereinander darstellt. In einer weiteren Ausbaustufe kann eine Elektronik vorgesehen werden, die unterschiedliche Auswertungen wie ein Differenzbild zur kontrastreicheren Darstellung erstellt. Anordnungen für diese Aufgaben werden in verschiedenen Varianten als Karten und Programme zur Bildverarbeitung angeboten.

Die Unempfindlichkeit gegen das Umgebungslicht gestattet es, gegenüber den bislang bekannten Systemen die Auswertung auch im Freien durchzuführen. Da die Stromaufnahme der CCD-Schwarz-Weiß-Kameras sehr niedrig liegt, kann sehr einfach, eine tragbare Variante der erfindungsgemäßen Vorrichtung zur Feststellung der Bläue geschaffen werden. Die Versorgung der Kameras 3, 4 kann z.B. aus einem handelsüblichen Laptop erfolgen, der seinerseits die Signale der Kamera(s) aufnimmt, die Berechnungen durchführt und auch ein Abspeichern der gewonnenen Informationen gestattet. Der Laptop kann dabei auch das in den Fig. dargestellte Auswertegerät 16 ausbilden. Damit kann auch eine Bewertung des einzukaufenden Rundholzes vor Ort, vor dem Transport, erfolgen.

## Patentansprüche

1. Verfahren zur Erkennung von Bläue bei Holz an einer Schnittfläche (2) eines Stammes (1), wobei von der Schnittfläche (2) reflektiertes Licht von zumindest einer bildgebenden Kamera (3) erfasst wird, **dadurch gekennzeichnet, dass** zumindest zwei Bilder in zumindest einem ersten und einem zweiten Wellenlängenbereich des elektromagnetischen Spektrums erfasst und die Bilder verglichen und ausgewertet werden.

2. Verfahren nach Anspruch 1, **dadurch gekennzeichnet, dass** der erste Wellenlängenbereich elektromagnetische Strahlung im für Menschen sichtbaren Bereich, vorzugsweise im blauen und/oder grünen Bereich des Lichtspektrums, und der zweite Wellenlängenbereich elektromagnetische Strahlung im Infrarotbereich abdeckt.

3. Verfahren nach Anspruch 1 oder 2, **dadurch gekennzeichnet, dass** die in den unterschiedlichen Wellenlängenbereiche erfassten Bilder vorzugsweise mittels eines Differenzverfahrens kombiniert werden.

4. Verfahren nach einem der Ansprüche 1 bis 3, **dadurch gekennzeichnet, dass** die Bilder unterschiedlicher Wellenlängenbereiche zeitlich nacheinander erfasst werden.

5. Verfahren nach Anspruch 4, **dadurch gekennzeichnet, dass** die Schnittfläche (2) abwechselnd über eine erste Strahlungsquelle (5) im ersten Wellenlängenbereich und über eine zweite Strahlungsquelle (6) im zweiten Wellenlängenbereich bestrahlt wird.

6. Verfahren nach einem der Ansprüche 1 bis 5, **dadurch gekennzeichnet, dass** die Schnittfläche (2) mit zumindest einer gleichzeitig den ersten Wellenlängenbereich und den zweiten Wellenlängenbereich abdeckenden Strahlungsquelle (7, 8) bestrahlt wird und die von der Schnittfläche (2) reflektierte Strahlung zumindest über eine für den ersten oder zweiten Wellenlängenbereich durchlässige Filtereinrichtung (F) geleitet wird.

7. Verfahren nach Anspruch 6, **dadurch gekennzeichnet, dass** die reflektierte Strahlung abwechselnd durch einen für den ersten Wellenlängenbereich durchlässigen ersten Filter (10) und einen für den zweiten Wellenlängenbereich durchlässigen zweiten Filter (11) der Filtereinrichtung (F) geleitet wird.

8. Verfahren nach einem der Ansprüche 1 bis 7, **dadurch gekennzeichnet, dass** die reflektierte Strahlung in einen ersten Strahlungsweg für Strahlung im ersten Wellenlängenbereich und einen zweiten Strahlungsweg für Strahlung im zweiten Wellenlängenbereich aufgeteilt wird und dass die Bilder im ersten Wellenlängenbereich durch eine erste Kamera (3) und im zweiten Wellenlängenbereich durch eine zweite Kamera (4) gleichzeitig erfasst werden.

9. Vorrichtung zur Erkennung von Bläue bei Holz an einer Schnittfläche (2) eines Stammes (1), mit zumindest einer bildgebenden Kamera (3) zur Erfassung von der Schnittfläche (2) reflektiertem Licht, **dadurch gekennzeichnet, dass** zumindest zwei Bilder in zumindest einem ersten und einem zweiten Wellenlängenbereich des elektromagnetischen Spektrums erfassbar sind und die Bilder einer Auswerteeinheit (16) zuführbar sind, wobei die Vorrichtung zumindest eine für elektromagnetische Strahlung im ersten oder zweiten Wellenbereich durchlässige Filtereinrichtung (F) im Strahlungsweg der reflektierten Strahlung zwischen der Schnittfläche (2) und der Kamera (3, 4) aufweist.

10. Vorrichtung nach Anspruch 9, **dadurch gekennzeichnet, dass** die Kamera (3, 4) ausgebildet ist um elektromagnetische Strahlung im ersten und zweiten Wellenlängenbereich zu erfassen, wobei vorzugsweise die Kamera (3, 4) durch eine Schwarz-Weiß-Digitalkamera gebildet ist.

11. Vorrichtung nach Anspruch 9 oder 10, **dadurch gekennzeichnet, dass** die Bilder unterschiedlicher Wellenlängenbereiche zeitlich nacheinander erfassbar sind.

12. Vorrichtung nach einem der Ansprüche 9 bis 11, **dadurch gekennzeichnet, dass** die Vorrichtung zumindest eine erste Strahlungsquelle (5) für Strahlung im ersten Wellenlängenbereich und zumindest eine zweite Strahlungsquelle (6) für Strahlung im zweiten Wellenlängenbereich aufweist, wobei die Schnittfläche (2) abwechselnd über die erste Strahlungsquelle (5) im ersten Wellenlängenbereich und über die zweite Strahlungsquelle (6) im zweiten Wellenbereich bestrahlbar ist.

13. Vorrichtung nach einem der Ansprüche 9 bis 12, **dadurch gekennzeichnet, dass** die vorzugsweise durch eine um eine Achse drehbares Filterrad (9) gebildete Filtereinrichtung (F) zumindest einen für den ersten Wellenlängenbereich durchlässigen ersten Filter (10) und zumindest einen für den zweiten Wellenlängenbereich durchlässigen zweiten Filter (11) aufweist, wobei die reflektierte Strahlung abwechselnd durch den ersten Filter (10) und den zweiten Filter (11) leitbar ist.

14. Vorrichtung nach einem der Anspruch 9 bis 13, **dadurch gekennzeichnet, dass** die Filtereinrichtung (F) als Strahlteiler ausgebildet ist, wobei die reflektierte Strahlung in einen ersten Strahlungsweg für Strahlung im ersten Wellenlängenbereich und einen zweiten Strahlungsweg für Strahlung im zweiten Wellenlängenbereich aufteilbar ist und dass zur Erfassung der Bilder im ersten Strahlungsweg eine erste Kamera (3) und im zweiten Strahlungsweg eine zweite Kamera (4) angeordnet ist.

15. Vorrichtung nach einem der Ansprüche 14, **dadurch gekennzeichnet, dass** der Strahlteiler durch einen selektiv beschichteten Spiegel (14) gebildet ist, welcher ausgebildet Strahlung im ersten Wellenlängenbereich zu reflektieren und Strahlung im zweiten Wellenlängenbereich durchzulassen.

16. Vorrichtung nach einem der Ansprüche 9 bis 15, **dadurch gekennzeichnet, dass** zumindest eine Kamera (3, 4), vorzugsweise samt Filtereinrichtung (F), zumindest teilweise von einem Tubus (13) umgeben ist.

17. Vorrichtung nach einem der Ansprüche 9 bis 16, **dadurch gekennzeichnet, dass** die optische Achse zumindest einer Kamera (3) etwa normal zur Schnittfläche (2) angeordnet ist.

18. Vorrichtung nach einem der Ansprüche 9 bis 17, **dadurch gekennzeichnet, dass** die Strahlungsquellen (5, 6) bezüglich der Schnittfläche (2) schattenfrei angeordnet sind.
